# EUROPEAN PATENT APPLICATION

(11) **EP 1 080 724 A1**
(43) Date of publication of application: **07.03.2001**
(21) Application number: 99913682.3
(22) Date of filing: 16.04.1999
(51) Int. Cl.: A61K 31/195, A61K 31/54, A61K 35/78

(54) **MTP ACTIVITY-LOWERING COMPOSITIONS**

(30) Priority: 22.05.1998 JP 14117698
(71) Applicant: Nippon Shinyaku Co., Ltd., Kyoto-shi Kyoto 601-8550 (JP)
(72) Inventor: YANAGITA, Teruyoshi, Saga-shi Saga 840-027 (JP); ANNO, Takahiko, Otsu-shi Shiga 520-0112 (JP)
(74) Representative: Pautex Schneider, Nicole
(86) International application number: JP9902023
(87) International publication number: WO9961015

(57) **Abstract**

Compositions which are useful in inhibiting the secretion of biological substances such as VLDL into the blood. These compositions are MTP activity-lowering compositions characterized by containing sulfur-containing amino acids such as cycloalliin and S-ethyl-L-cysteine or physiologically acceptable salts thereof.

## Description

### TECHNICAL FIELD

The present invention relates to sulfur-containing amino acids, represented by cycloalliin [(1S,3R,5S)-5-methyl-1,4-thiazane-3-carboxylic acid 1-oxide], and an extract containing the same.

### BACKGROUND ART

Naturally-occurring sulfur-containing amino acids, among which is cycloalliin, occur principally in plants of the genus Allium of the family Liliaceae, such as onion, garlic and scallion, in fairly large amounts. Among such naturally-occurring sulfur-containing amino acids, cycloalliin is known to have such pharmacologic activities as fibrinolytic, hypoglycemic and hypolipidemic activities [e.g. Atherosclerosis, 27, 347-351 (1977); JP Kokai H05-194237]. In other naturally-occurring sulfur-containing amino acids, too, the existence of certain pharmacologic activities such as platelet aggregation inhibitory activity, cholesterol-lowering activity and carcinostatic activity is known [Indian Journal of Experimental Biology, 34, 634-640 (1996) etc.].

Meanwhile, MTP (microsomal triglyceride transfer protein) is a heterodimer protein consisting of 58 KDa and 97 KDa subunits, which is known to be present in the endoplasmic reticulum lumen in the liver and small intestine and promote the transfer of neutral lipids, particularly triglyceride (TG), within the endoplasmic reticulum lumen [e.g. Ishigami et al.: Atherosclerosis, 24 (10), 533-540, 1997]. This MTP marshals the transferred TG and apo B to thereby form very-low-density lipoprotein (VLDL) particles, and this VLDL is secreted into the blood.

It is known that an excessive elevation of the blood VLDL level triggers various diseases associated with lipid metabolism [Fujioka et al. "Rinsho-Eiyo-no-Shimpo" (Advances in Clinical Nutrition), vol. 3, 81-91 (1993)]. For example, an excessive elevation of blood VLDL is said to promote atherosclerosis through hypoHDL(high-density lipoprotein)emia.

Therefore, should MTP activity, which is associated with the biosynthesis of VLDL, in the endoplasmic reticulum lumen in the liver, for instance, could be successfully controlled, the secretion of VLDL into the circulation would be suppressed to exert favorable effects on various diseases arising from an excessive elevation of the blood VLDL level.

### DISCLOSURE OF INVENTION

The object of the present invention is to provide a composition useful for suppressing the secretion of VLDL into the circulation.

After intensive investigations, the present inventors found that sulfur-containing amino acids, for example cycloalliin, which occur at high levels in plants of the genus Allium represented by onion (Allium cepa, L), have an MTP activity-lowering action and an apo B secretion-suppressing action, and have developed the present invention.

Thus, in one aspect, the present invention is directed to an MTP activity-lowering composition characterized by its comprising a sulfur-containing amino acid of the following general formula [I] or [Ia] (hereinafter referred to as the amino acid of the invention) 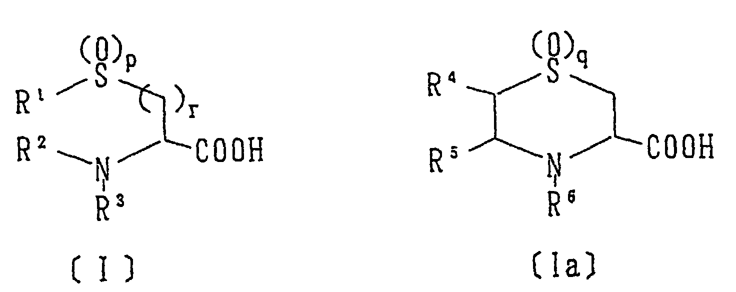 wherein R¹ represents H, alkyl or alkenyl, R², R³ may be the same or different and each represents H, alkyl or acyl, R⁴, R⁵ may be the same or different and each represents H or alkyl, R⁶ represents H, alkyl or acyl, p represents 0 or 1, q represents 0 or 1, and r represents an integer of 1-3, or a physiologically acceptable salt thereof.

An apo B antisecretory composition characterized by its comprising an amino acid of the invention or a physiologically acceptable salt thereof and a VLDL antisecretory composition characterized by its comprising an amino acid of the invention or a physiologically acceptable salt thereof are also subsumed in the concept of the present invention.

In the context of the present invention, the "alkyl" includes but is not limited to straight-chain or branched-chain alkyl groups of 1-6 carbon atoms, with straight-chain alkyl groups of 1-4 carbon atoms being preferred. As specific examples, there can be mentioned methyl, ethyl, propyl, isopropyl, butyl, isobutyl, sec-butyl, tert-butyl, pentyl, 1-methylbutyl, 2-methylbutyl, 3-methylbutyl, hexyl, 1-methylpentyl, 2-methylpentyl, 3-methylpentyl, 4-methylpentyl, 1-ethylbutyl, 2-ethylbutyl and 3-ethylbutyl.

In the context of the present invention, the "alkenyl" includes but is not limited to straight-chain or branched-chain alkenyl groups of 2-6 carbon atoms, with straight-chain alkenyl groups of 2-4 carbon atoms being preferred. As specific examples, there can be mentioned vinyl, 1-propenyl, isopropenyl, allyl, 1-butenyl, 2-butenyl, 1-pentenyl, 2-pentenyl, 3-pentenyl, 1-hexenyl, 2-hexenyl, 3-hexenyl and 4-hexenyl.

The "acyl" in the context of the present invention includes but is not limited to straight-chain or branched-chain acyl groups of 1-6 carbon atoms, among others, with straight-chain acyl groups of 2-4 carbon atoms being preferred. As specific examples, there can be mentioned formyl, acetyl, propionyl, butyryl, isobutyryl, valeryl, isovaleryl, pivaloyl and hexanoyl.

As preferred embodiments of the present invention, there can be mentioned an MTP activity-lowering composition characterized by its comprising an amino acid of the invention wherein R¹ is a straight-chain C₁₋₄ alkyl group or a straight-chain C₂₋₄ alkenyl group, R², R³ are the same or different and each is H or a straight-chain C₂₋₄ acyl group, R⁴ is H, R⁵ is a straight-chain C₁₋₄ alkyl group, R⁶ is H or a straight-chain C₂₋₄ acyl group, p is 0 or 1, q is 0 or 1 and r is 1 or 2 or a physiologically acceptable salt thereof, an apo B antiseoretory composition characterized by its comprising the above amino acid or physiologically acceptable salt, and a VLDL antisecretory composition characterized by its comprising the above amino acid or physiologically acceptable salt.

As the more preferred embodiments of the present invention, there can be mentioned an MTP activity-lowering composition characterized by its comprising an amino acid of the invention wherein R¹ is methyl, ethyl, propyl, butyl, allyl or 1-propenyl, R² is H, R³ is H or acetyl, R⁴ is H, R⁵ is methyl, ethyl, propyl or butyl, R⁶ is H or acetyl, p is 0 or 1, q is 0 or 1 and r is 1 or 2 or a physiologically acceptable salt thereof, an apo B antisecretory composition characterized by its comprising the above amino acid or physiologically acceptable salt, and a VLDL antisecretory composition characterized by its comprising the above amino acid or physiologically acceptable salt.

As preferred specific embodiments of the present invention, there may be mentioned an MTP activity-lowering composition characterized by its comprising an amino acid of the invention, that is cycloalliin, S-methyl-L-cysteine, S-ethyl-L-cysteine, S-propyl-L-cysteine, DL-methionine, DL-ethionine, S-methyl-L-cysteine sulfoxide, S-ethyl-L-cysteine sulfoxide, S-propyl-L-cysteine sulfoxide, or DL-methionine sulfoxide, or a physiologically acceptable salt thereof, an apo B antisecretory composition characterized by its comprising the above amino acid or physiologically acceptable salt, and a VLDL antisecretory composition characterized by its comprising the above amino acid or physiologically acceptable salt.

The physiologically acceptable salts include the corresponding salts with inorganic acids such as hydrochloric acid, sulfuric acid, nitric acid, phosphoric acid, hydrofluoric acid, hydrobromic acid; salts with organic acids such as acetic acid, tartaric acid, lactic acid, citric acid, fumaric acid, maleic acid, succinic acid, malic acid, malonic acid, methanesulfonic acid, ethanesulfonic acid, benzenesulfonic acid, p-toluenesulfonic acid, naphthalenesulfonic acid, camphorsulfonic acid; salts with alkali metals such as sodium, potassium; salts with alkaline earth metals such as calcium, magnesium; and aluminum salts.

Many species of the amino acid of the invention are known compounds and can be produced by the conventional technology or purchased from commercial sources. Cycloalliin, a species of the amino acid of the invention, can be produced by the technology described in, inter alia, Acta Chem. Scand., 13, 623 (1959); J. Org. Chem., 31, 2862 (1966); Chem. Pharm. Bull., 29(6) 1554-1560 (1981); and JP Kokai S55-40658.

The physiologically acceptable salt of the amino acid of the invention can also be easily synthesized by one skilled in the art starting with the amino acid of the invention. The hydrochloride, for instance, of the amino acid of the invention can be obtained by dissolving the amino acid of the invention in a solution of hydrogen chloride in an alcohol or ethyl acetate, while the sodium salt, for instance, of the amino acid of the invention can be obtained by mixing the amino acid of the invention with sodium hydroxide under stirring.

Furthermore, many species of the amino acid of the invention occur in plants of the genus Allium of the family Liliaceae, such as the onion, and, as such, can be extracted from such plants of the genus Allium by a suitable extraction technique and, where necessary, can be further purified. As plants of the genus Allium, there can be mentioned onion, garlic and scallion.

To mention a specific extraction technique, an onion extract, for instance, can be obtained by a process which comprises compressing raw onion bulbs with a suitable press and concentrating the juice or a process which comprises extracting raw onion bulbs with hot water or a solvent (e.g. ether, n-butanol) and distilling off the water or solvent. As the raw onion bulbs, the bulbs subjected to peeling, pasteurization and cutting to a suitable size can be employed. Before compression or extraction with hot water or the like, the onion bulbs may be heated to inactivate the CS lyase in the onion. In the case of hot water extraction, the onion may be pretreated with enzymes such cellulase, pectinase and protease in advance so as to improve the efficiency of juice extraction. The onion extract obtained in this manner contains the amino acid of the invention in a total concentration of about 0.1-0.3 weight %. With regard to individual species of the amino acid of the invention in the onion extract, cycloalliin accounts for about 0.02 weight %, S-methyl-L-cysteine sulfoxide accounts for about 0.02-0.05 weight %, and S-methyl-L-cysteine sulfoxide accounts for about 0.05-0.08 weight %.

For illustration, in order to enhance the cycloalliin content of the onion extract, the blanched onion juice or hot-water extract may be heat-treated at 90 120°C or treated with alkali at pH 8-12. These procedures may be followed in combination. Moreover, prior to such heat treatment and/or alkali treatment, the substrate material may be treated with an enzyme capable of cleaving the γ-glutamyl peptide, the cycloalliin precursor, in the onion (e.g. γ-glutamyl peptidase, γ-glutamyl transpeptidase, glutaminase). The cycloalliin can be isolated and purified from the onion extract by ion exchange chromatography.

The extract can be used as it is or after suitable processing, for example by adding an excipient such as dextrin or lactose to the extract and dehydrating the composition to give a powder.

Therefore, an MTP activity-lowering composition characterized by its comprising an extract containing the amino acid of the invention, an apo B antisecretory composition characterized by its comprising said extract, and a VLDL antisecretory composition comprising said extract are also subsumed in the concept of the present invention. The extract containing the amino acid of the invention includes an extract of a plant belonging to the genus Allium (an allium extract), among others, and said allium extract includes an onion extract, a garlic extract and a scallion extract, among others.

The composition according to the present invention (hereinafter referred to as the composition of the invention) can be applied to animals inclusive of humans in the form of a pharmaceutical composition or a health food-like food composition.

The dosage of the composition of the invention varies with the recipient's factors such as age and body weight and the blood VLDL level but usually the composition of the invention can be orally taken within the range of 1-5000 mg as the amino acid of the invention or a physiologically acceptable salt thereof per diem per adult human. The preferred dose range is 100-2000 mg and the more preferred range is 200-1500 mg. Depending on cases, a lower dose will be sufficient or conversely a higher dose may be needed. As to the daily dosing schedule, the composition of the invention is preferably taken in 2-5 divided doses. The composition of the present invention can be taken any hour of the day, i.e. before each meal, between meals, or after each meal. It may be taken even during a meal.

The composition of the invention may contain the amino acid of the invention, a physiologically acceptable salt thereof or an extract containing the amino acid of the invention either as it is alone or as formulated in a physiologically acceptable nontoxic, inert carrier within the range of, for example, 0.01%-99.5%, preferably 0.5%-90%.

As the carrier mentioned above, a solid, semisolid or liquid diluent, filler and other formulating agents can be used either singly or combinedly. The composition of the invention can be supplied in all kinds of forms such as neat powders, capsules, tablets, sugar-coated tablets, granules, powders, suspensions, solutions, syrups and drops. In certain cases, it may be provided in the form of an injection.

### BEST MODE FOR CARRYING OUT THE INVENTION

The following test examples are further illustrative of the present invention.

### Test Example 1 MTP activity-lowering activity

SD rats weighing about 200 g in groups of 6 were given 20 mg or 60 mg of the hydrochloride of the amino acid of the invention in 10 ml of water in 3 divided doses daily for 2 weeks. Thereafter, the liver was excised and homogenized using a homogenizing buffer (50 mM Tris (pH 7.4), 50 mM potassium chloride, 1.5 mH EDTA, leupeptin (5 µg/ml) and 2 mM phenylmethylsulfonyl fluoride) and the homogenate was centrifuged to give a liver homogenate. This liver homogenate was adjusted to a protein concentration of about 2 mg/mL with the same homogenizing buffer and 1 part of deoxycholate solution (0.56%, pH 7.5) was added to 10 parts of the homogenate. The reaction was then carried out at 4°C for 30 minutes, whereby MTP was liberated from the microsomal fraction. The membrane fraction was then removed by centrifugation to give a supernatant. This supernatant was diluted with Tris buffer (a buffer solution containing 15 mM Tris (pH 7.4), 40 mM NaCl, 1 mM EDTA, 0.02% NaN₃) and dialyzed against the same buffer at 4°C overnight. The dialysate thus obtained was analyzed for MTP activity by the method of J. R. Wetterau [Science, 258, 999 (1992)]. The results are shown in Table 1.

**Table 1**

| MTP activity values | |
|---|---|
| | MTP activity (%) |
| Control group | 9.09±1.0 |
| Cycloalliin HCl, 20 mg | 6.12±0.7* |
| Cycloalliin HCl, 60 mg | 4.92±1.0* |
| S-methyl-L-cysteine HCl, 60 mg | 5.68±0.6* |
| S-methyl-L-cysteine sulfoxide HCl, 60 mg | 5.43±0.6* |

| | |
|---|---|
| *: p<0.05 (Duncan multiple comparison test) | |

It is clear from Table 1 that the above salt of the amino acid of the invention causes a significant decrease in MTP activity.

### Test Example 2 Apo B antisecretory activity (1)

HepG2 cells (100×10⁴ cells/dish) were sown on 3.5 cm dishes and precultured using 1 ml DME (containing 10% FCS) medium per dish. Following this preculture, the cells were washed with DME medium twice, added to the test medium, and cultured at 37°C under 95% air-5% carbon dioxide for 24 hours. The test medium was prepared by dissolving cycloalliin in dimethyl sulfoxide and diluted with DME medium containing 1% BSA and 0.5 mM oleic acid to a final cycloalliin concentration of 10⁻⁶ M or 10⁻⁴ M.

After 24 hours' culture, the medium was recovered and the amount of apo B secreted into the medium was determined by ELISA. Thus, anti-human apo B antibody (a 100-fold dilution) was distributed into a 96-well ELISA plate, 100 µL per well, and incubated at 37°C for 2 hours. Then, 100 µL/well of Block Ace was added and the plate was further incubated at 37°C for 2 hours. The wells were washed with PBS-Tween × 3 and 100 µL each of apo B standard solution and HepG2 culture were added, followed by further incubation at 4°C for 16 hours. After the wells were washed with PBS-Tween × 3, 100 µL/well of anti-human apo B marker antibody (a 200-fold dilution) was added and the plate was incubated at 37°C for 2 hours. The wells were washed with PBS-Tween × 3, then 100 µL/well of the substrate solution was added, and the plate was incubated at 25°C for 30 minutes. The reaction was stopped by adding 50 µL of 4.5 M H₂SO₄ and the absorbance at 482 nm was measured to estimate the amount of apo B secretion. The results are shown in Table 2.

**Table 2**

| Apo B secretions | |
|---|---|
| | Apo B secretions (µg/well) |
| Control group | 9.92±0.80 |
| Cycloalliin, 10⁻⁶ M | 7.54±1.13* |
| Cycloalliin, 10⁻⁴ M | 7.67±1.31* |

| | |
|---|---|
| *: p<0.01 (Duncan multiple comparison test) | |

It is apparent from Table 2 that cycloalliin causes a significant suppression of apo B secretion.

### Test Example 3 Apo B antisecretory activity (2)

HepG2 cells were cultured in DME medium supplemented with 1% BSA and 0.5 mM oleic acid for 24 hours. Then, the medium was changed to 1% BSA-DME medium containing 10 µM of the amino acid of the invention and 0.5 µci of [¹⁴C] acetic acid was simultaneously added. The cells and medium were recovered after 24 hours or 48 hours of culture. The recovered cells were homogenized by sonication, and after quantitation of protein, the amount of apo B secretions was determined by ELISA. The results are shown in Table 3.

**Table 3**

| Apo B secretions | | |
|---|---|---|
| | Apo B secretions (ng/mg protein) | |
| | After 24 hr of culture (%) | After 48 hr of culture (%) |
| Control group | 2544±425 (100) | 2080±110 (100) |
| S-methyl-L-cysteine | 2511±173 (98.7) | 1906±122 (91.6) |
| S-ethyl-L-cysteine | 2390±615 (93.9) | 994±111 (47.8) |
| S-n-propyl-L-cysteine | 2157± 86 (84.8) | 555± 23 (26.7) |

### Test Example 4 TG antisecretory activity

HepG2 cells were cultured in DME medium supplemented with 1% BSA and 0.5 mM oleic acid for 24 hours. Then, the medium was changed to 1% BSA-DME containing 10 µM of the amino acid of the invention and simultaneously 0.5 µci of [¹⁴C] acetic acid was added. The cells and medium were recovered after 24 hours of culture. The recovered cells were homogenized by sonication, and after quantitation of protein, the amount of secreted triglyceride (TG) was determined from the amount of labeled lipid in the medium. The results are shown in Table 4.

**Table 4**

| TG secretion | |
|---|---|
| | TG secretions ([¹⁴C]dpm × 10⁻²/mg protein) |
| Control group | 161.2± 2.2 |
| S-methyl-L-cysteine | 128.2±14.6 |
| S-ethyl-L-cysteine | 102.2± 7.1 |
| S-n-propyl-L-cysteine | 99.0± 9.3 |
| S-n-propyl-L-cysteine sulfoxide | 105.8±18.9 |
| DL-methionine sulfoxide | 72.1± 8.5 |

### EFFECT OF INVENTION

The composition of the invention is capable of lowering MTP activity which is associated with VLDL biosynthesis and inhibiting secretion of apo B as well, so that it is effective in suppressing the secretion of VLDL into the blood.

## Claims

1. An MTP activity-lowering composition characterized by its comprising a sulfur-containing amino acid of the following general formula [I] or [Ia] 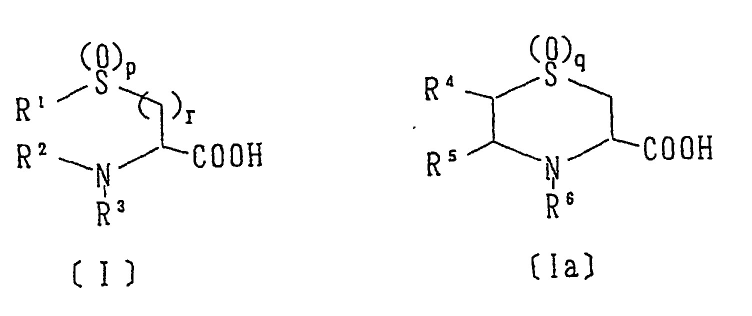 wherein R¹ represents H, alkyl or alkenyl, R², R³ may be the same or different and each represents H, alkyl or acyl, R⁴, R⁵ may be the same or different and each represents H or alkyl, R⁶ represents H, alkyl or acyl, p represents 0 or 1, q represents 0 or 1, and r represents an integer of 1-3, or a physiologically acceptable salt thereof.

2. An MTP activity-lowering composition characterized by its comprising a sulfur-containing amino acid of the general formula [I] or [Ia] in Claim 1 wherein R¹ is a straight-chain C₁₋₄ alkyl group or a straight-chain C₂₋₄ alkenyl group, R², R³ are the same or different and each is H or a straight-chain C₂₋₄ acyl group, R⁴ is H, R⁵ is a straight-chain C₁₋₄ alkyl group, R⁶ is H or a straight-chain C₂₋₄ acyl group, p is 0 or 1, q is 0 or 1 and r is 1 or 2 or a physiologically acceptable salt thereof.

3. An MTP activity-lowering composition characterized by its comprising a sulfur-containing amino acid of the general formula [I] or [Ia] in Claim 1 wherein R¹ is methyl, ethyl, propyl, butyl, allyl or 1-propenyl, R² is H, R³ is H or acetyl, R⁴ is H, R⁵ is methyl, ethyl, propyl or butyl, R⁶ is H or acetyl, p is 0 or 1, q is 0 or 1 and r is 1 or 2 or a physiologically acceptable salt thereof.

4. An MTP activity-lowering composition characterized by its comprising a sulfur-containing amino acid of the general formula [I] or [Ia] in Claim 1, which is cycloalliin, S-methyl-L-cysteine, S-ethyl-L-cysteine, S-propyl-L-cysteine, DL-methionine, DL-ethionine, S-methyl-L-cysteine sulfoxide, S-ethyl-L-cysteine sulfoxide, S-propyl-L-cysteine sulfoxide, or DL-methionine sulfoxide, or a physiologically acceptable salt thereof.

5. An MTP activity-lowering composition characterized by its comprising an extract containing the sulfur-containing amino acid defined in any of Claims 1-4.

6. An MTP activity-lowering composition as claimed in Claim 4 wherein the extract containing the sulfur-containing amino acid defined in any of Claims 1-4 is an extract of a plant of the genus Allium.

7. An MTP activity-lowering composition as claimed in Claim 5 wherein the extract of a plant of the genus Allium is an onion extract, a garlic extract or a scallion extract.

8. An apo B antisecretory composition characterized by its comprising the sulfur-containing amino acid or physiologically acceptable salt defined in Claim 1.

9. An apo B antisecretory composition characterized by its comprising the sulfur-containing amino acid or physiologically acceptable salt defined in Claim 2.

10. An apo B antiseeretory composition characterized by its comprising the sulfur-containing amino acid or physiologically acceptable salt defined in Claim 3.

11. An apo B antisecretory composition characterized by its comprising the sulfur-containing amino acid or physiologically acceptable salt defined in Claim 4.

12. An apo B antisecretory composition characterized by its comprising an extract containing the sulfur-containing amino acid defined in any of Claims 1-4.

13. An apo B antisecretory composition as claimed in Claim 12 wherein the extract containing the sulfur-containing amino acid defined in any of Claims 1-4 is an extract of a plant of the genus Allium.

14. An apo B antisecretory composition as claimed in Claim 13 wherein the extract of a plant of the genus Allium is an onion extract, a garlic extract or a scallion extract.

15. A VLDL antisecretory composition characterized by its comprising the sulfur-containing amino acid or physiologically acceptable salt defined in Claim 1.

16. A VLDL antisecretory composition characterized by its comprising the sulfur-containing amino acid or physiologically acceptable salt defined in Claim 2.

17. A VLDL antisecretory composition characterized by its comprising the sulfur-containing amino acid or physiologically acceptable salt defined in Claim 3.

18. A VLDL antisecretory composition characterized by its comprising the sulfur-containing amino acid or physiologically acceptable salt defined in Claim 4.

19. A VLDL antisecretory composition characterized by its comprising an extract containing the sulfur-containing amino acid defined in any of Claims 1-4.

20. A VLDL antisecretory composition as claimed in Claim 19 wherein the extract containing the sulfur-containing amino acid defined in any of Claims 1-4 is an extract of a plant of the genus Allium.

21. A VLDL antisecretory composition as claimed in Claim 20 wherein the extract of a plant of the genus Allium is an onion extract, a garlic extract or a scallion extract.
